# EUROPEAN PATENT APPLICATION

(11) **EP 1 308 147 A2**
(43) Date of publication of application: **07.05.2003**
(21) Application number: 02024483.6
(22) Date of filing: 30.10.2002
(51) Int. Cl.: A61F 13/15

(54) **Apparatus and method for manufacturing disposable wearing articles**

(30) Priority: 02.11.2001 JP 2001337363
(71) Applicant: Zuiko Corporation, Osaka 566-0045 (JP)
(72) Inventor: Yoshinari, Tanaka, Osaka 564-0032 (JP); Yuzu, Ichiura, Osaka 567-0845 (JP)
(74) Representative: Schwabe - Sandmair - Marx

(57) **Abstract**

An apparatus for manufacturing wearing articles, comprises a supplier (1) for supplying a continuous body (W) having the ability to absorb a liquid; a cutter (2) for cutting the continuous body (W) at a length in the flow direction shorter than the width of the continuous body (W); and a drum (3) for changing the orientation of the cut pieces (N) of the continuous body (W) so that the cut pieces (N) are elongate in the flow direction.

## Description

### FIELD OF THE INVENTION

The present invention relates to a method for manufacturing disposable wearing articles.

### BACKGROUND OF THE INVENTION

When disposable wearing articles and the like are manufactured from a web that is continuous in the direction of flow of the process, the web is first cut, and then another web is placed on the cut web while the cut web is being transported.

If the web is cut to obtain pieces that are elongate in the width direction of the web, the subsequent process may find difficulty in placing another member on the cut web. On the contrary, if a narrow web is cut to obtain pieces that are elongate in the flow direction, the production efficiency (yield) decreases due to the use of the narrow web because the rotational speed of a drum for transporting the web is limited.

In view of the above, an object of the present invention is to provide a method for manufacturing disposable wearing articles capable of improving the production efficiency.

Absorbers for disposable wearing articles are produced by pulverizing a material such as pulp and allowing the pulverized matter to be adsorbed to a pattern drum. If the rotational speed of the drum is increased in an attempt to enhance the production efficiency, suction of air into a number of pores formed on the pattern drum becomes more difficult. This causes a decrease in the flow rate of air, and therefore blocks a smooth flow of the pulverized matter toward a mesh. The resultant absorbers fail to have a predetermined volume.

In view of the above, another object of the present invention is providing a method for manufacturing disposable wearing articles capable of providing absorbers having a predetermined volume even when the rotational speed of a pattern drum is increased.

### SUMMARY OF THE INVENTION

To attain the objects described above, the first method of the present invention is a method for manufacturing wearing articles, including the steps of: supplying a continuous body having a predetermined thickness and the ability to absorb a liquid; cutting the continuous body at a length in the flow direction shorter than the width of the continuous body; and changing the orientation of the cut pieces of the continuous body so that the cut pieces are elongate in the flow direction.

Examples of the disposable wearing articles manufactured according to the present invention include paper diapers, paper pants, sanitary napkins and pads for incontinence. The articles also include blanks of these products.

In the first method described above, in the supplying step, a web unwound from a web roll may be supplied to a cutter roller, or a continuous web formed with a pattern drum may be supplied to the cutter roller. The cutting of the web includes both cutting the web to separate into pieces and cutting out pieces of a predetermined shape from the web.

The orientation of the cut web pieces may be changed while the pitch of the cut web pieces is being widened with a repitch turn drum, or the orientation may be changed after the widening of the spacing between the cut web pieces.

The second method of the invention is a method for manufacturing disposable wearing articles, including the steps of: pulverizing a web material to obtain pulverized matter; depositing the pulverized matter like cotton on a plurality of adsorbing portions of a pattern drum while rotating the pattern drum to obtain absorbers, the adsorbing portions adsorbing the pulverized matter with a negative pressure of air; and changing the orientation of the absorbers with respect to the flow direction.

In the second method, the orientation of the absorbers also may be changed while the spacing between the adjacent absorbers is being widened with a repitch turn drum, or the orientation may be changed after the widening of the spacing between the absorbers.

The third method of the invention is a method for manufacturing disposable wearing articles, including the steps of: pulverizing an absorber material to obtain pulverized matter; depositing the pulverized matter like cotton on a plurality of adsorbing portions of a pattern drum while rotating the pattern drum to obtain absorbers, the adsorbing portions adsorbing the pulverized matter with a negative pressure of air; and increasing the pitch of the absorbers in the flow direction.

In the second and third methods, pulp is generally used as the absorber material and is pulverized to obtain fluff pulp. Each absorber constitutes a core for absorbing a body fluid. A polymer absorber may be blended into the fluff pulp.

In the third method, the absorber may be placed between a back sheet and a top sheet after the orientation changing step or the pitch increasing step. The absorber may be covered with another sheet (tissue paper) before being placed between the back and top sheets.

### BRIEF DESCRIPTION OF THE DRAWINGS

FIG. **1** is a schematic layout of an example of a manufacturing apparatus according to a first aspect of the present invention.
FIG. **2A** is a perspective view demonstrating a repitch and turn method, and FIG. **2B** is a schematic side view of an example of a repitch turn device.
FIG. **3** is a schematic layout of another example of the manufacturing apparatus according to the first aspect of the present invention.
FIG. **4** is a schematic layout of an example of a manufacturing apparatus according to a second aspect and a third aspect of the present invention.
FIGS. **5A** and **5B** are schematic perspective views of a pattern drum, illustrating examples of a pattern, and FIG. **5C** is a perspective view of another example of the pattern (adsorbing portion).
FIG. **6** is a schematic layout of an example of a manufacturing apparatus according to the second and third aspects of the present invention.

### DESCRIPTION OF PREFERRED EMBODIMENTS

Hereinafter, preferred embodiments of the present invention will be described with reference to the accompanying drawings.

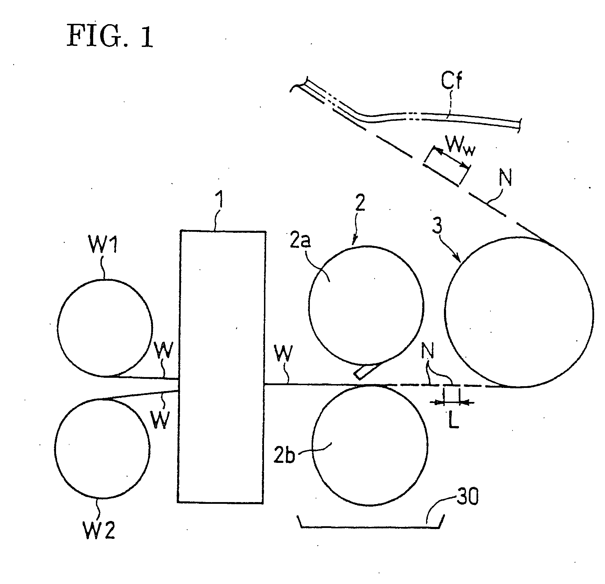

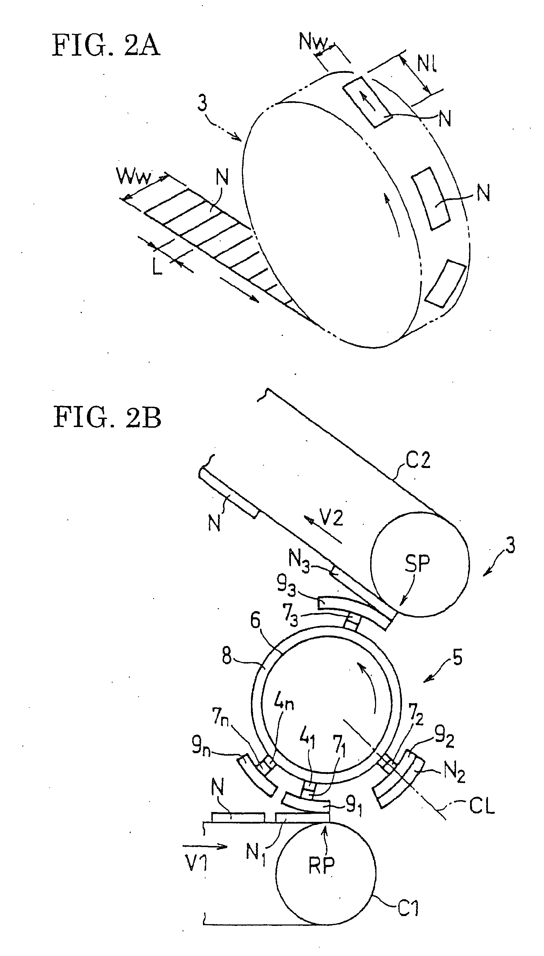

FIGS. **1** and **2** illustrate the first embodiment of the invention. Referring to FIG. **1,** when web rolls W1 and W2 are those of an absorber, the web has a predetermined thickness. Due to this thickness, the total length of the web wound into the web roll W1 or W2 is comparatively short. Therefore, frequent change of the web rolls W1 and W2 is required.

The system shown in FIG. **1** includes: a joint unit **1** for joining the first web roll W1 and the second web roll W2; a cutter unit **2** for cutting the web from the web roll W1 or W2 at a predetermined length; and a repitch turn device **3** for changing the orientation of articles (cores, absorbers) N such as cut absorbers.

For the joint unit **1**, a known method such as that disclosed in Japanese Laid-Open Patent Publication No. 11-268152 may be employed, to realize continuous supply of the web W. That is, one web roll W1 or W2 can be changed to the other new web roll W2 or W1 while the web roll W1 or W2 is still in use. The first and second web rolls W1 and W2 preferably should be in the same shape and structure.

The joint unit **1** receives the web W from the first web roll W1. When detecting that the first web roll W1 is running low, the joint unit **1** changes the web roll as will be described later, to supply the web W from the second web roll W2 in place of the first web roll W1. The joint unit **1** includes a joint portion, a dancer portion (dancer roller) and a sensor although these components are not shown. The sensor may monitor the diameter of the web roll or monitor the rotational speed of the web roll, to detect that the web roll is running low. Otherwise, the sensor may count the time passing from the start of the unwinding of the web roll to detect the remaining amount of the web W of the web roll.

For the change of the web roll, the dancer portion previously loosens (buffers) the web W from the first web roll W1. When the sensor detects that the first web roll W1 is running low, the loosened part of the web W is released. During this release, the speed of the unwinding of the first web roll W1 can be lowered to zero or at least to a speed at which the joint can be changed from the first web roll W1 to the second web roll W2. The joint unit **1** then changes the joint by cutting the web W of the first web roll W1 and joining the top end of the web W of the second web roll W2 to the cut end.

The cutter unit **2** includes a cutter roller **2a** and a chopping roller **2b**. With rotation of the rollers **2a** and **2b**, the web W is cut at a predetermined length. The length L (in the flow direction) at which the web is cut is shorter than the width Ww of the web W as shown in FIG. **2A**. Cut articles N are then transported to the repitch turn device **3.**

As shown in FIG. **2B,** the repitch turn device **3** includes a repitch turn drum **5** and first and second conveyors **C1** and **C2**. The first conveyor **C1** is placed upstream of the drum **5,** while the second conveyor **C2** is placed downstream of the drum **5**. The first conveyor **C1** transports the articles N with a narrow spacing therebetween (at a small pitch).

For turning the articles N, various structures such as that disclosed in Japanese Laid-Open Patent Publication No. 63-317576 may be adopted. Otherwise, a novel structure described below, for example, may be adopted.

The drum **5** includes a rotation portion **6**, a guide **8,** and a plurality of sets of a slide **4**_{**i**}, a turner **7**_{**i**} and a pad **9**_{**i**}. The rotation portion **6** rotates continuously in the direction of transportation of the articles N. The guide **8** rotates together with the rotation portion **6.** The slides **4**_{**i**} slide on the guide **8** so that the spacing therebetween varies along the rotation direction. The turners **7**_{**i**} turn around the normal CL of the drum **5**.

The pads **9**_{**i**} have a number of suction pores for adsorption of the articles N. A negative pressure is formed inside the pads **9**_{**i**} in the positions shown by pads **9**_{**1**} to **9**_{**3**}, to thereby allow the articles N to be adsorbed to the pads **9**_{**i**}.

When coming close to a receiving position RP, the pad **9**_{**i**} rotates at a circumferential velocity V1 that is roughly identical to the speed of the first conveyor **C1** until it passes the receiving position RP. When coming close to a supply position SP, the pad **9**_{**i**} rotates at a circumferential velocity V2 that is roughly identical to the speed of the second conveyor **C2** until it passes the supply position SP. The circumferential velocities V1 and V2 have the relationship of V2 > V1.

Once an article **N**_{**1**} is transported to the receiving position RP with the first conveyor **C1**, the article **N**_{**1**} is adsorbed to the pad **9**_{**1**}. The pad **9**_{**1**}, which has received the article N₁ at the receiving position RP, rotates at a gradually increasing speed toward the supply position SP at which the pad **9**_{**3**} is releasing an article **N**_{**3**}. At the supply position SP, the pad **9**_{**3**} has stopped adsorption of the article **N**_{**3**}, to enable the second conveyor **C2** to easily receive the article **N**_{**3**} by adsorption.

The pad **9i** is turnably fitted to the slide **4i** via the turner **7i**. Therefore, the pad **9i** turns by a predetermined angle (for example, 90° ) around the normal CL during movement from the receiving position RP to the supply position SP, to change the orientation of the article **N**_{**i**}. By this turning, as shown in FIG. **2A,** the length NI of article **N**_{**i**} in the flow direction is longer than the width Nw thereof.

By changing the orientation of the articles N as described above, it becomes possible to use a wide web W. This reduces the frequency of the change of the web roll, and also increases the total amount of the web W unwound. Therefore, the production efficiency improves substantially.

The angle by which the pad **9**_{**i**} is turned for change of the orientation is not necessarily 90° , but instead may vary over a range of angle, as may be desired.

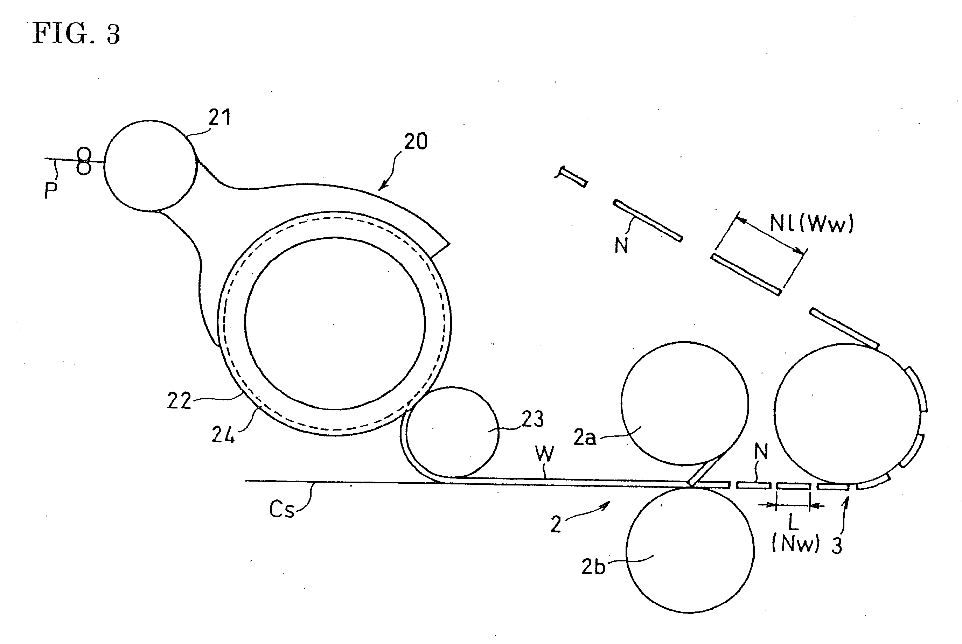

FIG. **3** illustrates a manufacturing apparatus of the second embodiment of the present invention. The apparatus includes a core generator **20**, a cutter unit **2** and a repitch turn device **3**. A web W is formed by defibrating (pulverizing) pulp P with a defibrator (pulverizer) **21** to obtain fluff pulp and forming a cotton-like deposition of the fluff pulp alone or the fluff pulp with synthetic fibers and/or highly absorptive polymer particles (polymer absorber) blended therein, to be used as absorptive cores N. The core generator **20** includes the defibrator **21** for defibrating the pulp and a pattern drum **22**. The defibrated fibers are deposited on the pattern drum **22**, to form the web W as the continuous body. The pattern drum **22** has an adsorbing portion **24** made of a mesh on the surface. The inside of the drum **22** is vacuumed with a negative pressure during rotation of the drum **22**, so that the continuous web W is formed continuously along the surface profile of the mesh **24.**

An example of the method for manufacturing the cores N will be described below. The adsorbing portion **24** of the pattern drum **22** is put in an inwardly vacuumed state with a vacuum fan not shown. The material pulp P unwound from a pulp roll not shown is defibrated with the defibrator **21**, and the defibrated fluff pulp is blown toward the pattern drum **22**. During this blowing, the pattern drum **22** is vacuumed with a negative pressure, to thereby form the web W having a thickness.

The web W formed in the manner described above is supplied to the cutter unit **2** via a transfer roller **23**. The cutter unit **2** cuts the web W at a predetermined length in the flow direction, which is shorter than the width Ww of the web W. The cut cores N are sent to the repitch turn device **3**. The repitch turn device **3** turns the orientation of the pads, for example, by 90° , as in the previous embodiment, so that the length NI of the cores N in the flow direction is longer than the width Nw thereof (in the direction crossing the flow, see FIG. **2A**)

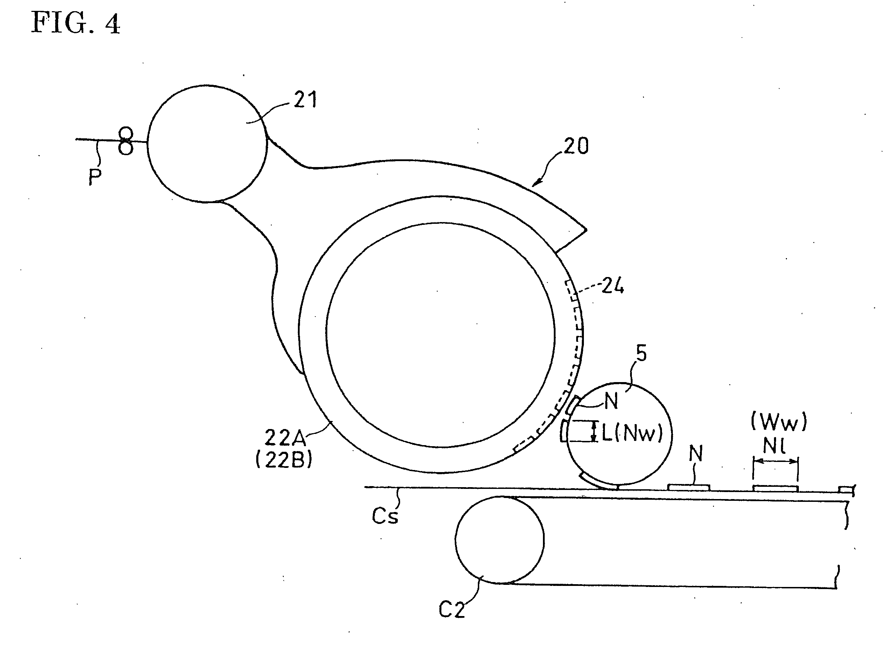

FIG. **4** illustrates a manufacturing apparatus in accordance with the third embodiment of the present invention. This apparatus includes a core generator **20**, a repitch turn drum **5** and a conveyor **C2.** In this embodiment, the core generator **20** has a plurality of adsorbing portions **24**, placed all around the circumference of a pattern drum **22A** (**22B**) with roughly equal spacing therebetween, although in FIG. **4** only part of the adsorbing portions **24** are shown.

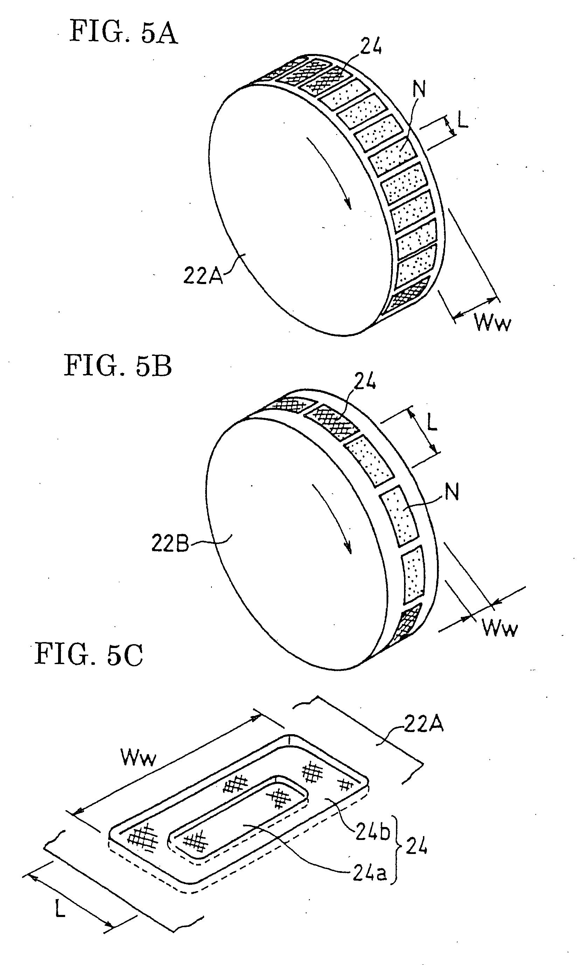

The core generator **20** includes the pattern drum **22A**, which is shown in more detail in FIG. **5A.** The plurality of adsorbing portions **24** are placed on the circumference of the pattern drum **22A** apart from each other in the circumferential direction. As shown in FIG. **5C,** each of the adsorbing portions **24** has a recess made of a mesh on which defibrated fibers and the like are deposited.

The adsorbing portion **24** has a length Ww in the width direction of the pattern drum **22A** that is longer than a length L in the rotation direction of the pattern drum **22A**. The cores N produced by deposition by the core generator **20** are sent to the repitch turn drum **5** shown in FIG. **4.**

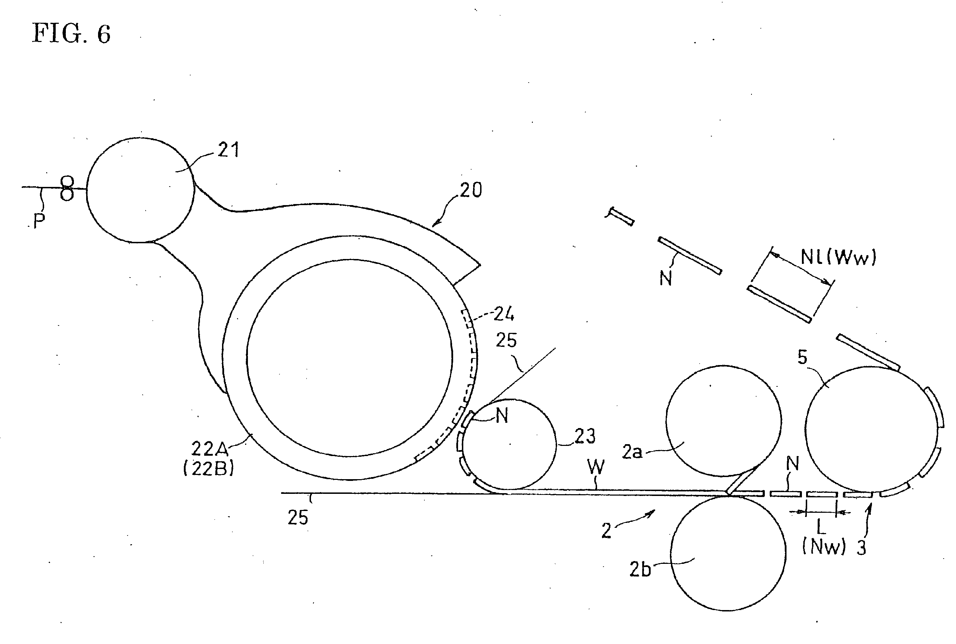

The cores N may be enclosed with at least one cover sheet **25** before entering the repitch turn drum **5** to obtain a continuous body (FIG. **6**). The cutter unit **2** may cut the cover sheet **25** between the adjacent cores N to transfer the enclosed cores N to the repitch turn.

The repitch turn drum **5** turns the orientation of the pads as in the previous embodiments, so that the length NI of the cores N in the flow direction is longer than the width Nw thereof in the direction crossing the flow. During the change of the orientation, the inter-pad spacing may be widened to widen the spacing between the cores N.

The conveyor **C2** may receive the absorbers N from the drum **5** via a carrier sheet Cs such as tissue paper and a liquid-impermeable sheet, as illustrated in FIG.4.

FIG. **5B** illustrates another example of the pattern drum, denoted by **22B**.

In the pattern drum shown in FIG. **5A,** the length Ww of the adsorbing portions **24** in the direction crossing the pattern drum **22A** is longer than the length L thereof in the rotation direction of the pattern drum **22A** (transverse stretch). Using this type of pattern drum, during the deposition of the pulverized matter, a plurality of cores N elongate in the width direction are sequentially produced on the circumference of the pattern drum **22A**. During the change of the orientation, these cores N are changed to the state elongate in the flow direction.

The pattern of the pattern drum **22B** shown in FIG. **5B** is the opposite (longitudinal stretch), in which the length L of the adsorbing portions **24** is longer than the width Ww thereof. Using this type of the pattern drum, during the deposition of the pulverized matter, the cores N elongate in the rotation direction are sequentially produced on the circumference of the pattern drum **22B**.

In comparison of the patterns in FIGS. **5A** and **5B**, the pattern in FIG. **5A** is generally superior to the pattern in FIG. **5B** for the following reasons. The area of the mesh portions per unit area is large in the former pattern. Therefore, a smooth flow of air is ensured, and thus the pulverized matter can be easily deposited even when the pattern drum **22A** rotates at high speed. Also, since a larger flow rate of air is ensured in the pattern in FIG. **5A** than in the pattern in FIG. **5B**, three-dimensional deposition of fibers and the like is possible. In addition, even when the pattern drums **22A** and **22B** have the same rotational speed and the same pitch of the adsorbing portions **24**, the transportation speed increases once the orientation of the cores N is changed by the repitch turn drum **5** in the stage subsequent to the core generator **20** shown in FIG. **4**, and thus the speed increases also in the stage subsequent to the repitch turn drum **5**. Therefore, from the aspect of productivity, the pattern in FIG. **5A** is superior to the pattern in FIG. **5B**.

If the rotational speed of the pattern drums **22A** and **22B** is low, the pattern in FIG. **5B** may be used. In this case, a repitch drum for only changing the spacing between the cores N may be used in place of the repitch turn drum.

Fibers may be deposited three-dimensionally. For example, a first part **24a** of the adsorbing portion **24** may be recessed from a second part **24b** toward the axis of the pattern drum **22A**. In reverse, the second part **24b** may be made deeper than the first part **24a.**

The first and second parts **24a** and **24b** are made of a mesh, for example, on which defibrated fibers are deposited due to suction from inside.

As shown in FIG. **1**, the cut cores N or articles N may be covered with a plurality of anti-leak walls (three-dimensional material) Cf. Also, the system may include a dust collector **30** shown in FIG. 1, for collecting by suction sawdust generated during the cutting of the web W with the cutter unit **2**.

As described above, according to the first method of the present invention, the cut web pieces are changed in orientation after the cutting to be elongate in the flow direction. Therefore, a wide web can be used as the original web, and thus the frequency of change of the web roll can be reduced. In addition, with a wide web to be unwound, the total amount (total area) of the unwound web increases. With the reduced frequency of change of the web roll and the increased total amount of the unwound web, the production efficiency significantly increases.

In the above method, if the web is formed using a pattern drum, the effect described below in relation to the second method is also obtained.

According to the second method of the present invention, the orientation of the absorbers is changed. Therefore, if wide absorbers are to be changed in orientation to be elongate in the flow direction, it is possible to increase the number of adsorbing portions and the area of the mesh portions on one pattern drum. With an increase of the area of the mesh portions per unit area, the flow rate of air increases. This enables smooth flow of the pulverized matter toward the drum, and thus absorbers having a predetermined volume can be obtained.

With the smooth flow of the pulverized matter, absorbers having a predetermined volume can be obtained even when the rotational speed of the pattern drum is increased. Therefore, the production efficiency significantly improves.

With the adsorbing portions elongate in the width direction of the pattern drum placed on the pattern drum, a number of absorbers can be produced during one rotation of the pattern drum. This further improves the production efficiency.

According to the third method of the present invention, the pitch of the absorbers is widened after the absorbers have been formed by deposition on the pattern drum. This makes it possible to increase the number of absorbers formed on the pattern drum. Therefore, as in the second method, the flow of air is made smooth, and thus absorbers having a predetermined volume can be obtained. Also, the production efficiency improves.

## Claims

1. A method for manufacturing wearing articles, comprising:
supplying a continuous body having a predetermined thickness and the ability to absorb a liquid;
cutting the continuous body at a length in a flow direction shorter than a width of the continuous body, resulting in a plurality of cut pieces having an orientation in which the cut pieces are elongate in a direction generally transverse to the flow direction; and
changing the orientation of the cut pieces of the continuous body so that the cut pieces are elongate in the flow direction.

2. The method of claim 1, further comprising the step of increasing a pitch of the cut pieces of the continuous body in the flow direction.

3. The method of claim 1, wherein the step of supplying a continuous body comprises: defibrating pulp to obtain fluff; and depositing the fluff to obtain the continuous body.

4. The method of claim 1, wherein the step of supplying a continuous body comprises: defibrating pulp to obtain fluff; depositing the fluff to a plurality of adsorbing portions placed on a pattern drum to obtain a plurality of cores; and enclosing the plurality of cores with at least one cover sheet to obtain the continuous body.

5. The method of claim 3, wherein depositing the fluff comprises adsorbing the fluff and a polymer absorber to an adsorbing portion placed on a pattern drum.

6. A method for manufacturing wearing articles, comprising the steps of:
defibrating pulp to obtain fluff;
depositing the fluff on a pattern drum having a plurality of adsorbing portions to obtain absorbers, a length of the absorbers in a width direction of the pattern drum being longer than a length of the absorbers in a circumferential direction of the pattern drum wherein the absorbers have an orientation such that the absorbers are elongate in a direction generally transverse to flow direction; and
changing the orientation of the absorbers so that the absorbers are elongate in the flow direction.

7. An apparatus for manufacturing wearing articles, comprising:
means for supplying a continuous body having an ability to absorb a liquid;
means for cutting the continuous body at a length in a flow direction shorter than a width of the continuous body; and
means for changing an orientation of the cut pieces of the continuous body so that the cut pieces are elongate in the flow direction.

8. The apparatus of claim 7, wherein the means for supplying a continuous body supplies the continuous body by joining a first web unwound from a first web roll with a second web unwound from a second web roll.

9. The apparatus of claim 7, wherein the means for supplying a continuous body comprises: a defibrator for defibrating pulp to obtain fluff; and a pattern drum on which the fluff is deposited to form the continuous body.

10. A method for manufacturing disposable wearing articles, comprising:
pulverizing an absorber material to obtain pulverized matter;
depositing the pulverized matter on a plurality of adsorbing portions of a pattern drum while rotating the pattern drum, to obtain absorbers, the adsorbing portions adsorbing the pulverized matter with a negative pressure of air; and
increasing a pitch of the absorbers in the flow direction.

11. The method of claim 10, wherein a length of the adsorbing portions in a rotation direction of the pattern drum is longer than a length of the adsorbing portions in a width direction of the pattern drum, and in depositing the pulverized matter, the plurality of absorbers elongate in the rotation direction are produced along a circumference of the pattern drum.

12. The method of claim 4, wherein depositing the fluff comprises adsorbing the fluff and a polymer absorber to a plurality of adsorbing portions placed on a pattern drum.
